# EUROPÄISCHE PATENTANMELDUNG

(11) **EP 2 873 965 A1**
(43) Veröffentlichungstag der Anmeldung: **20.05.2015**
(21) Anmeldenummer: 13192614.9
(22) Anmeldetag: 13.11.2013
(51) Int. Cl.: G01N 21/53, B01D 1/22, B01D 3/08, C12M 1/21, G01N 29/14, G01N 21/85

(54) **Vorrichtung und Verfahren zur Erkennung einer Schaumentwicklung**

(71) Anmelder: Büchi Labortechnik AG, 9230 Flawil (CH)
(72) Erfinder: Bürki, Martin, 9248 Bichwil (CH)
(74) Vertreter: Hepp Wenger Ryffel AG

(57) **Zusammenfassung**

Die Erfindung betrifft eine Vorrichtung (1) zur Erkennung einer Schaumentwicklung in einem Prozessgefäss, umfassend einen Detektor (3) zum Erfassen eines elektromagnetischen und/oder eines akustischen Signals. Dabei ist die Vorrichtung (1) in einem Prozessgefäss anordenbar ist. Die Erfindung betrifft weiter ein Verfahren zur Erkennung einer Schaumentwicklung in einem Prozessgefäss.

## Beschreibung

Die Erfindung betrifft eine Vorrichtung und ein Verfahren zur Erkennung einer Schaumentwicklung gemäss den Oberbegriffen der unabhängigen Ansprüche.

Beim Betrieb gängiger Verdampfungssysteme wird ein zu verdampfendes Medium durch Erhitzen eines Prozessgefässes aus der Fest- und/oder Flüssigphase in die Dampfphase überführt. In vielen Fällen wirft das zu verdampfende Medium Blasen auf bzw. ein Schaum entwickelt sich auf der Oberfläche des Mediums. Dies ist zumeist abhängig vom zu verdampfenden Medium und von angelegten Verfahrensparametern. Vorrichtungen zur Erkennung einer Schaumentwicklung werden beim Eindampfen, Verdampfen und Destillieren von Flüssigkeiten, Flüssigkeitsgemischen, Stoffen und Stoffgemischen eingesetzt. Dabei ist es erforderlich eine aufkommende Schaumentwicklung zu erkennen und gegebenenfalls zu verhindern oder ein Überschäumen eines Schaums zu verhindern. Es soll vermieden werden, dass Schaum in eine Dampfdurchführung oder in einen Auffangbehälter eines Destillats eintritt. Insbesondere beim Betrieb eines Rotationsverdampfers, ist eine Schaumentwicklung zu vermeiden, da durch das Überschäumen und das Eintreten des Schaums in den Auffangbehälter das Destillat verunreinigt wird, bzw. Stoffe des Rückstandes verloren gehen.

Vorrichtungen und Verfahren zur reinen Vermeidung und zur Erkennung mit darauf folgenden Massnahmen zur Reduktion einer Schaumentwicklung sind bekannt. Verfahren und Vorrichtungen zur Vermeidung einer Schaumentwicklung nutzen beispielsweise vordefinierte Zugabeintervalle eines zu verdampfenden Mediums in einen Verdampferkolben, womit eine Schaumentwicklung vermieden wird. Dies ist in US 2003/0111184 A1 gezeigt. Optische Schaumsensoren zur Erkennung einer Schaumentwicklung sind in DE 10 2010 007 559 A1 beschrieben. Dabei ist ein optisches System bestehend aus einer Lichtquelle und einem Sensor in einem Fenster angeordnet, welches in einer Wand eines Bioreaktorbehälters eingelassen ist. Bei Erkennung eines aufkommenden Schaums mittels eines reflektierten Kontrollsignals werden Antischaumagenzien, welche der Schaumentwicklung entgegen wirken, zugegeben.

Vorgenannte Vorrichtung, welche auf einer optischen Erkennung einer Schaumentwicklung basiert, verwendet jedoch spezielle Behälter, insbesondere mit einer speziellen Behälterwand. Dabei müssen ein oder mehrere Fenster und/oder Öffnungen in der Wand des Behälters eingelassen sein, in welche Elemente eines optischen Systems eingelassen sind, um eine optische Erkennung durchführbar zu machen. Während des Betriebs kann es zu Ablagerungen und Verschmutzungen der Fenster und/oder Öffnungen kommen, was die Erkennung einer Schaumentwicklung nachteilig beeinträchtigt. Zudem ist eine Verwendung des Systems mit rotierenden Behältern eingeschlossen.

Es ist daher die Aufgabe der Erfindung die Nachteile des Standes der Technik zu überwinden. Insbesondere ist es Aufgabe der Erfindung, eine Vorrichtung und ein Verfahren zur Erkennung einer Schaumentwicklung in einem Prozessgefäss bereitzustellen, welche geringe Herstellungskosten aufweisen, welche ein kostengünstiges Nachrüsten von Systemen, insbesondere von Verdampfungssystemen, ermöglichen und welche eine verbesserte Leistungsfähigkeit aufweisen.

Diese Aufgaben werden durch die in den unabhängigen Patentansprüchen definierten Merkmale gelöst.

Die Erfindung betrifft eine Vorrichtung zur Erkennung einer Schaumentwicklung in einem Prozessgefäss, umfassend einen Detektor zum Erfassen eines elektromagnetischen und/oder eines akustischen Signals. Die Erfindung zeichnet sich dadurch aus, dass die Vorrichtung in einem Prozessgefäss anordenbar ist. Auf diese Weise wird ein kostengünstiges Nachrüsten von Systemen gewährleistet, da die Vorrichtung zur Erkennung einer Schaumentwicklung im Prozessgefäss angeordnet und innerhalb dieses betrieben werden kann. Somit ist kein speziell an die Schaumerkennung angepasstes Prozessgefäss, z.B. mit einem Fenster und/oder einer Öffnung in der Gefässwand, erforderlich. Zudem kann eine Schaumentwicklung mittels des Detektors innerhalb des Prozessgefässes also nahe beim Entstehen erkannt werden. Eine Verschmutzung der Gefässwand hat somit keinen negativen Einfluss.

Die Vorrichtung kann zusätzlich eine Quelle zum Erzeugen des elektromagnetischen und/oder des akustischen Signals aufweisen. Damit kann ein vordefiniertes elektromagnetisches und/oder akustisches Signal generiert werden, welches mittelbar und/oder unmittelbar vom Detektor innerhalb des Prozessgefässes detektiert wird. Die Vorrichtung ist somit unabhängig von externen oder zusätzlichen Quellen. Die Zuverlässigkeit der Schaumerkennung wird somit verbessert.

Das von der Quelle erzeugte, insbesondere gepulste Signal kann für das menschliche Auge und/oder das menschliche Gehör nicht wahrnehmbar sein. Das Signal kann auf der Verwendung von Infrarotlicht basieren. Dabei weist die Quelle ein Sendefenster beispielsweise im Bereich von 850 nm auf. Der Detektor weist einen Empfangsbereich auf, welcher das Sendefenster umfasst, insbesondere zwischen 730 bis 1010 nm. Selbstverständlich kann das Signal als elektromagnetische Welle, insbesondere im Radiofrequenzbereich (Funkwellen) verschiedener Frequenzbänder, oder im Hochfrequenzbereich, insbesondere im Bereich 0,1 bis 100 GHz, ausgesendet werden. Selbstverständlich kann das Signal auch als Ultraschall ausgesendet werden. Durch die Verwendung eines gepulsten Signals kann das Signal-Rausch-Verhältnis (signal-to-noise-ratio) verbessert werden.

Durch die Verwendung eines schmalbandigen Signals mit einem entsprechenden Filter vor dem Detektor kann das Signal-Rausch Verhältnis (signal-to-noise) verbessert werden.

Ausserdem kann das Signal auf der Seite der Quelle moduliert und auf der Seite des Detektors entsprechend demoduliert werden, was ebenfalls zu einem verbesserten Signal-Rausch Verhältnis (signal-to-noise-ratio) führt.

Schliesslich kann ein quasizufälliges Signal (spread spectrum) verwendet werden. In Kombination mit einem Korrelator nach dem Detektor wird wiederum das Signal-Rausch Verhältnis verbessert und die Störfestigkeit gegenüber Fremdsignalen verbessert.

Anwendungsspezifische Filter oder Vorfilter, die dem Detektor vorgelagert oder nachgeschaltet sind, können auf das charakteristische zufällige Reflexionsverhalten eines Schaums optimiert sein. Dabei ist die einfachste Ausführung die Verwendung eines Hochpassfilters.

Bei der Quelle kann es sich um eine Leuchtdiode zum Aussenden von schmalbandigem Licht (LED, light-emitting-diode), einen Infrarot-Transmitter, eine Laserdiode, einen Piezolautsprecher oder einen piezoelektrischen Quarz- oder Keramikschwinger handeln. Entsprechend wäre der Detektor beispielsweise als Photodiode, Phototransistor, Photowiderstand (LDR, light dependent resistor), Infrarotempfänger oder Piezosensor (Richtmikrophon) ausgestaltet. Der Detektor kann als Kombination mit einem kapazitivem Detektor ausgestaltet sein, beispielsweise als Kombination aus Infrarot-Detektor und kapazitivem Detektor.

Selbstverständlich können auch eindimensionale und/oder zweidimensionale (1D und 2D) Sensoren, beispielsweise CCD (charge-coupled device) Bildsensoren zur Erkennung von Bewegung im Prozessgefäss umfasst sein. Dabei weisen vorzugsweise die Bildsensoren Bereiche mit ausreichender Tiefenschärfe auf, um das Erkennen einer Bewegung, insbesondere einer Schaumentwicklung, zu erfassen.

Die Vorrichtung kann eine Einheit aufweisen, in welcher die Quelle und der Detektor angeordnet sind. Dabei kann die Einheit als eine einzelne Baugruppe ausgestaltet sein, wobei Kombinationssensoren verwendet werden können. Innerhalb der Einheit können die Quelle und der Detektor nebeneinander oder voneinander beabstandet angeordnet sein. Somit können, im Falle dass die Quelle und der Detektor nebeneinander positioniert sind, vom Schaum reflektierte Signale detektiert werden. Sind die Quelle und der Detektor beabstandet voneinander angeordnet, vorzugsweise beabstandet in einem Bereich von 1 bis 5 cm werden überwiegend durch den Schaum transmittierte Signale detektiert. Letzteres setzt voraus, dass ein Bereich der Vorrichtung, welcher entweder die Quelle oder den Detektor umfasst, in einer Zone des Prozessgefässes, in welcher sich Schaum befindet oder in welcher sich Schaum entwickeln wird, positioniert ist.

Die Einheit der Vorrichtung kann stabförmig ausgestaltet sein, insbesondere gekapselt in einem für das Signal transparenten Stab, insbesondere aus Glas oder Kunststoff, angeordnet sein. Auf diese Weise kann die Einheit optimal innerhalb eines Prozessgefässes, insbesondere innerhalb eines länglich ausgestalteten Prozessgefässes, positioniert werden und/oder durch weiterführende Bauteile, insbesondere bei einem Verdampfungssystem, wie beispielsweise eine Dampfdurchführung, hindurchreichen. Die im Stab gekapselte Einheit ist vorteilig dahingehend, dass sie ein einfaches Reinigen der Einheit ermöglicht. Beispielsweise ist die Einheit in ein für das Signal transparentes Rohr, bevorzugt ein Glasrohr oder einem Kunststoffrohr, eingeschoben. Zudem reinigt rückfliessendes Kondensat die gekapselte Einheit.

Die Quelle und der Detektor können derart relativ zueinander angeordnet sein, dass der Detektor ein von einem Schaum reflektiertes Signal und/oder ein durch den Schaum transmittiertes Signal detektiert. Auf diese Weise können Signale, die nach Interaktion mit dem Schaum reflektiert oder transmittiert werden, vom Detektor erfasst werden. Anhand des empfangenen Signals wird so eine Schaumentwicklung erkannt. Vorgenannter Schaum kann von einem Medium aus einem Stoff oder einem Stoffgemisch und/oder einer Flüssigkeit oder einem Flüssigkeitsgemisch, insbesondere von einem Lösungsmittel oder einem Lösungsmittelgemisch umfassend gängige Lösungsmittel, herrühren.

Beispielhafte Lösungsmittel, welche üblicherweise in Verdampfungssystemen benutzt werden, sind Acteon, Benzol, Chlorbenzol, 1,2-Dichlorethan, Dichlormethan, Diethylether, Dioxan, Essigsäu-re, Ethanol, Ehylacetat, Heptan, Hexan, Methanol, Pentan, n-Propylalkohol, Tetrachlorethylen, Toluol, Trichlorethylen, Trichlormethan, Wasser und Xylole. Weitere gängige Lösungsmittel sind dem Fachmann bekannt.

Vor dem Detektor kann eine Blende, ein Tubus, eine Optik und/oder einen Filter zum Abschirmen und Filtern unerwünschter Streusignale angeordent sein. Ebenso ist die Verwendung von Gittern und/oder Masken zusätzlich oder alternativ möglich. Eine solche Anordnung ist auch vor der Quelle denkbar. Auf diese Weise können das empfangene Signal und/oder das ausgesendete Signal anforderungsgemäss gefiltert werden. Insbesondere eine Detektion von Streustrahlung und unerwünschte Reflexionen werden verringert. Dies resultiert in einem verbesserten Signal-Rausch-Verhältnis. Ausserdem kann bei reflektiven Systemen ein direktes, transmittives Übersprechen von der Quelle zum Detektor verhindert werden.

Bevorzugt kann die Quelle als eine Infrarot-LED (light-emitting-diode) einen Abstrahlwinkel von 30° (±15°) aufweisen. Entsprechend handelt es sich bei dem Detektor um einen Phototransistor mit Blende und Ifrarot-Filter.

Die Vorrichtung kann zusätzlich eine Auswerteinheit zum Auswerten des vom Detektor erfassten Signals umfassen. Die Vorrichtung stellt somit schon das ausgewertete Signal zur Steuerung bzw. zur Weiterverarbeitung zur Verfügung. Anwendungsübliche Auswerteinheiten umfassen einen Verstärker sowie einen Korrelator. Mittels des Verstärkers wird das Signal auf einen Amplitudenwert angehoben, so dass dessen weitere Bearbeitung einfach realisierbar ist. Der Korrelator filtert einen Störanteil des Signals weg. Alternativ kann ein Hochpassfilter, ein Tiefpassfilter oder ein Bandpassfilter, je nach verwendetem Signaltyp, verwendet werden. In der einfachsten Ausführung wird dieses Signal mittels eines Komparators digitalisiert, so dass lediglich die An-/Abwesenheit von Schaum dargestellt wird. Selbstverständlich sind auch komplexere Auswerteinheiten denkbar. Vorzugsweise ist die Auswerteinheit auf einer einzigen Leiterplatte, insbesondere auf derselben Leiterplatte wie der Detektor angeordnet.

Zur Datenübertragung des ausgewerteten Signals kann eine diskrete Signalleitung oder aber auch eine andere Form der Datenübertragung vorgesehen sein. Entsprechend umfasst die Vorrichtung eine Datenschnittstelle zur Übertragung des ausgewerteten Signals.

Die Vorrichtung kann zusätzlich einen Temperatursensor zum Erfassen einer Temperatur im Prozessgefäss und/oder in einem Verdampfungssystem umfassen. Die erfasste Temperatur kann zur Steuerung oder zur Reglung des Betriebs eines Verdampfungssystems berücksichtigt werden, indem prozessspezifische Parameter auf vordefinierte Werte eingestellt werden. Prozessspezifische Parameter eines Verdampfungssystems umfassen eine Temperatur einer Heizvorrichtung, eine Rotationsgeschwindigkeit des Prozessgefässes und/oder einen angelegten Druck im System. Auf diese Weise kann das Verdampfungssystem auf optimale Betriebsbedingungen zum Verdampfen eines Mediums eingestellt werden. Ein zusätzlicher separater Temperatursensor wird nicht benötigt.

Die Erfindung betrifft weiter die Verwendung einer Vorrichtung, wobei die Vorrichtung in einem Prozessgefäss angeordnet ist. Die Vorrichtung ist vorstehend beschrieben. Durch die Verwendung der Vorrichtung innerhalb des Prozessgefässes kann ein Schaum und/oder eine Schaumentwicklung direkt im Prozessgefäss erkannt und frühzeitig reagiert werden. Störeinflüsse werden somit reduziert.

Ein weiterer Aspekt der Erfindung betrifft ein Verdampfungssystem, insbesondere einen Rotationsverdampfer, umfassend ein Prozessgefäss und eine Vorrichtung zur Erkennung einer Schaumentwicklung mit einem Detektor zum Erfassen eines elektromagnetischen und/oder akustischen Signals. Dabei ist die Vorrichtung im Prozessgefäss anordenbar oder angeordnet. Auf diese Weise wird eine Schaumentwicklung während des Betriebs des Verdampfungssystems, insbesondere während des Verdampfens und/oder Eindampfens von Stoffen oder Stoffgemischen im Verdampfungssystem, erkannt. Die Vorrichtung kann wie vorgängig beschrieben ausgestaltet sein.

Dabei kann zusätzlich ein Temperatursensor im Verdampfungssystem, insbesondere in einem Prozessgefäss, angeordnet sein. Eine erfasste Temperatur des Prozessgefässes kann für die Regelung angelegter Prozessparameter des Verdampfungssystems, insbesondere einer Temperatur einer Heizvorrichtung, einer Rotationsgeschwindigkeit des Prozessgefässes und/oder eines angelegten Drucks, berücksichtigt werden.

Die Vorrichtung kann statisch oder mit dem Verdampferkolben rotierend angebracht sein. Bei einer bevorzugten Anbringung ist die Vorrichtung an einem statischen Element des Verdampfungssystems, insbesondere des Rotationsverdampfers, angebracht und kann durch eine Dampfdurchführung bis in ein Prozessgefäss hinein reichen. Auf diese Weise können Verdampfungssysteme, insbesondere Rotationsverdampfer, mit einer erfindungsgemässen Vorrichtung nachgerüstet werden.

Das Verdampfungssystem kann Mittel aufweisen, um Schaum im Prozessgefäss zu reduzieren. Vorgenannte Mittel umfassen eine Vorrichtung zur Erhöhung des Drucks im Prozessgefäss, zum Senken einer Prozesstemperatur, insbesondere der Temperatur einer Heizvorrichtung, zum Verändern der Rotationsgeschwindigkeit eines Prozessgefässes und/oder zum Eingeben eines Antischaummittels. Durch die vorgenannten Mittel bzw. Massnahmen kann ein erkannter Schaum reduziert werden.

Ein weiterer Aspekt der Erfindung betrifft ein Verfahren zum Erkennen einer Schaumentwicklung in einem Prozessgefäss, insbesondere eines Verdampfungssystems, mit einer Vorrichtung zur Schaumerkennung oder eines Verdampfungssystems. Die Vorrichtung oder das Verdampfungssystem können wie vorgängig beschrieben ausgestaltet sein. Dabei umfasst das Verfahren die Schritte
i) Erfassen eines elektromagnetischen und/oder akustischen Signals mittels eines im Prozessgefäss angeordneten Detektors der Vorrichtung, und
ii) Auswerten des erfassten Signals nach vorgegebenen Kriterien zur Erkennung einer Schaumentwicklung.

Durch das Anordnen der Vorrichtung im Prozessgefäss kann ein Schaum und/oder eine Schaumentwicklung direkt im Prozessgefäss erkannt und frühzeitig reagiert werden. Störeinflüsse werden reduziert. Die Kriterien umfassen beispielsweise Veränderungen des Signals und/oder der Signalstärken, insbesondere des Sendeimpulses und/oder Ausblenden von regelmässigen Störungen.

Das Verfahren kann den Schritt des Aussendens eines elektromagnetischen und/oder akustischen Signals von einer Quelle, welche im Prozessgefäss angeordnet ist, umfassen. Auf diese Weise kann ein ausgesendetes Signal, welches von Schaum und/oder einem aufkommenden Schaum verändert wird, durch den Detektor der Vorrichtung erfasst werden. Die Detektion von Schaum ist dadurch unabhängig von einer externen Quelle und entsprechend sind weniger Störeinflüsse vorhanden.

Die Quelle kann dabei ein, insbesondere gepulstes, Signal im Infrarotbereich, insbesondere in einem Wellenlängenbereich von 700 bis 1400 nm, insbesondere im Bereich von 850 nm, und/oder Schallwellen, insbesondere Ultraschallwellen im Frequenzbereich von 20 kHz bis 1 GHz ausstrahlen. Der Detektor weist einen Empfangsbereich auf, welcher den Bereich des ausgesendeten Signals umfasst, und liegt beispielsweise im Bereich von 730 bis 1010 nm. Durch die Verwendung von gepulsten Signalen wird das Signal-Rausch-Verhältnis (signal-to-noise-ratio) verbessert. Selbstverständlich kann das Signal als elektromagnetische Welle, insbesondere im Radiofrequenzbereich (Funkwellen) verschiedener Frequenzbänder, oder im Hochfrequenzbereich, insbesondere im Bereich 0,1 bis 100 GHz, ausgesendet werden. Der Detektor ist entsprechend anzupassen. Bei der Quelle kann es sich um eine Leuchtdiode zum Aussenden von schmalbandigem Licht (LED, light-emitting-diode), einen Infrarot-Transmitter, eine Laserdiode, einen Piezolautsprecher oder einem piezoelektrischen Quarz- oder Keramikschwinger, und bei dem Detektor um eine Photodiode, einen Phototransistor, einen Photowiderstands (LDR, light dependent resistor), einen Infrarotempfänger oder einen Piezosensor handeln.

Der Detektor kann das von der Quelle ausgesendete und vom Schaum im Prozessgefäss durch Reflexion und/oder Transmission veränderte Signal detektieren. Auf diese Weise können Signale mittels des Detektors nach Reflexion an einem Medium, insbesondere an aufkommendem Schaum, detektiert werden.

Das Verfahren kann weitere Schritte zur Reduktion eines Schaums bei Anwesenheit von Schaum umfassen, wie beispielsweise eine Druckerhöhung im Prozessgefäss und/oder eine Reduktion einer Prozesstemperatur, insbesondere der Temperatur im Prozessgefäss und/oder der Temperatur in einer Kühlvorrichtung, und/oder eine Veränderung der Bewegungsparameter des Prozessgefässes und/oder ein Einleiten eines Antischaummittels.

Die Erfindung wird im Folgenden anhand von Abbildungen exemplarischer Ausführungsbeispiele näher erläutert. Es zeigen:
- Figur 1:: Eine schematische Darstellung einer erfindungsgemässen Vorrichtung,
- Figur 2:: Eine Schaumerkennung mittels der Vorrichtung aus Figur 1,
- Figur 3a:: Perspektivische Darstellung eines Rotationsverdampfers mit der Vorrichtung im Schnitt entlang einer Achse
- Figur 3b:: Vergrösserte Darstellung der Anbringung der Vorrichtung.

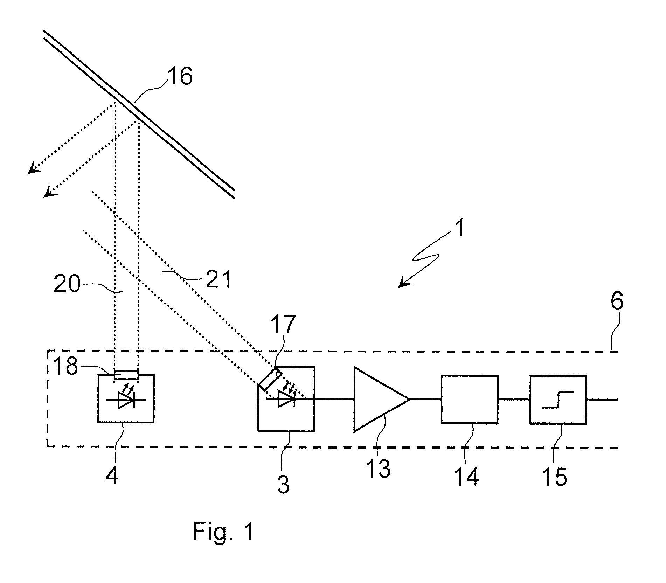

Figur 1 zeigt schematisch eine erfindungsgemässe Vorrichtung 1 umfassend eine Quelle 4, in der Form einer LED, einen Detektor 3 in der Form einer Photodiode, einen Verstärker 13, einen Korrelator 14, und einen Komparator 15. Alternativ können die Elemente Verstärker 13, Korrelator 14 und Komparator 15 in einem internen Schaltkreis integriert und/oder deren Funktion mittels einer Software erfüllt werden. Die Vorrichtung 1 ist gekapselt in einem Glasstab 6 angeordnet und in einem Prozessgefäss anordenbar oder angeordnet. Der Quelle 4 ist eine Blende 18 vorgelagert, welche ein Aussenden eines definiert konzentrierten Signals 20, insbesondere eines Lichtstrahls, ermöglicht. Alternativ kann der Quelle 4 zusätzlich ein Filter und/oder eine Linsenoptik und/oder eine Spiegeloptik vorgelagert sein. Dem Detektor 3 ist eine Empfangsbegrenzung 17 vorgelagert.

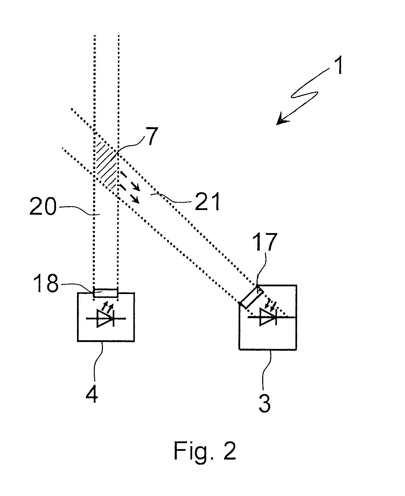

Alternativ kann dem Detektor 3 zusätzlich einer Blende und/oder einem Filter und/oder einer Linsenoptik und/oder einer Spiegeloptik vor-gelagert sein. Die Empfangsbegrenzung 17 grenzt ein einfallendes Signal derart ein, dass die Detektion von Streustrahlung verringert wird, und fokussiert gegebenenfalls ein ankommendes Signal. Das von der Quelle 4 ausgesendete Signal 20, welches an einer Wand 16 des Prozessgefässes reflektiert wird, gelangt aufgrund gewählter geometrischer Einschränkungen nicht in den Detektor 3. Vielmehr wird das Signal in einen dem Detektor 3 abgewandten und entfernten Bereich geleitet. Trifft das von der Quelle 4 ausgesendete Signal 20 in einem Überschneidungsbereich mit einem in den Detektor einfallenden Signalweg 21 auf ein Medium 7 (vgl. Fig. 2), insbesondere auf einen Schaum, so wird das Signal an diesem Medium 7 reflektiert und kann vom Detektor 3 erfasst werden. Vom Detektor 3 erfasste Signale werden in den Verstärker 13 geleitet und verstärkt. Das verstärkte Signal kann direkt einem Komparator zugeführt werden, oder wird wie dargestellt vorgängig mittels eines Korrelators 14 oder eines Hochpasses gefiltert. Der Korrelator 14 und/oder der Komparator 15 können eine dynamische Schwelle aufweisen, welche beispielsweise eine Verschmutzung des Glasstabes kompensiert. Eine weitere Verarbeitung des Signals kann mittels einer Elektronik, welche ebenfalls im Glasstab integriert oder extern angeordnet sein kann, erfolgen. Anhand einer ermittelten Schaumerkennung können Schritte automatisiert und/oder manuell eingeleitet werden, die einer Schaumentwicklung entgegenwirken. Diese Schritte umfassen beispielsweise eine Druckerhöhung im Prozessgefäss, eine Reduktion einer Prozesstemperatur, insbesondere der Temperatur einer Heizvorrichtung, eine Veränderung der Rotationsgeschwindigkeit des Prozessgefässes und/oder ein Einleiten eines Antischaummittels Figur 2 zeigt eine Schaumerkennung mittels der Vorrichtung 1 gemäss Figur 1, wobei lediglich die Quelle 4 und der Detektor 3 dargestellt sind. Die Quelle 4 sendet Signal 20 aus, welches durch die Blende 18 zu einem gerichtetem Strahl geformt wird. Dieses kann insbesondere in Form von gepulstem Infrarotlicht, vorzugsweise mit einer Wellenlänge von 850 nm, ausgesendet werden. Das ausgesendete Signal 20 trifft in einem Überschneidungsbereich mit einem in den Detektor einfallenden Signalweg 21 auf ein Medium 7, insbesondere auf einen Schaum. Das Signal 20 wird am Medium 7, insbesondere am Schaum, reflektiert und derart abgelenkt, dass es in den Detektor 3 gelangt. Dabei wird das in den Detektor 3 einfallende Signal von der vorgelagerten Empfangsbegrenzung 17 eingegrenzt. Der Detektor 3 weist einen Empfangsbereich von 730 bis 1010 nm auf. Anhand des erfassten Signals lässt sich eine Schaumentwicklung erkennen. Zudem lassen sich eine oder mehrere Differenzmessungen durchführen, um das Umgebungslicht und andere Störeinflüsse zu subtrahieren. Diese Referenzwerte können berücksichtigt werden und für die Auswertung der sich mit der Zeit verändernden Signaleigenschaften bei Schaumentwicklung herangezogen werden. Die Überwachung der Schaumentwicklung kann an einem oder mehreren Zeitpunkten oder kontinuierlich während eines Verdampfungsprozesses erfolgen.

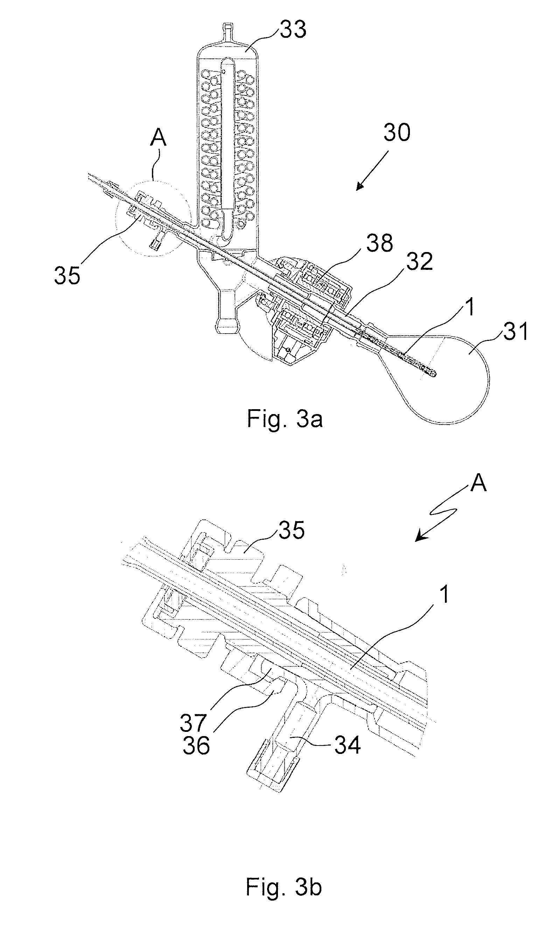

Figur 3a zeigt einen Rotationsverdampfer 30 umfassend ein Verdampfungsgefäss 31, eine Dampfdurchführung 32 und einen Flüssigkeitskühler 33, einen Einsatz 35 und eine erfindungsgemässe Vorrichtung 1. Die Vorrichtung ist gekapselt in einem Stab, insbesondere einem durchsichtigem Stab, ausgestaltet. Die Vorrichtung 1 ist an einer Seite des Flüssigkeitskühlers 33 mittels des Einsatzes 35 (vgl. Figur 3b) am Rotationsverdampfer 30 statisch angebracht. Die Vorrichtung 1 reicht durch den Flüssigkeitskühler 33 und die Dampfdurchführung 32 bis in das Verdampfungsgefäss 31 hinein. Der Ausschnitt A zeigt die Elemente des Einsatzes 35 (vgl. Figur 3b).

Figur 3b zeigt die Anbringung der Vorrichtung 1 am Rotationsverdampfer 30 als vergrösserte Darstellung des Ausschnittes A (vgl. Figur 3a). Der Einsatz 35 ist an dem Rotationsverdampfer 30 angebracht und weist eine Aussparung, in welche die gekapselte Vorrichtung 1 eingeführt ist, auf. Der Einsatz 35 ist mittels eines Haltelements 36, welches um ein Haltemittel 37 des Rotationsverdampfers 30 (vgl. Figur 3a) greift, am Rotationsverdampfer fixiert. Die gekapselte Vorrichtung 1 kann innerhalb des Einsatzes 35 ausgerichtet und mittels eines Klemmelements 34, welches den Einsatz 35 einklemmt, innerhalb des Einsatzes fixiert werden.

## Patentansprüche

1. Vorrichtung (1) zur Erkennung einer Schaumentwicklung in einem Prozessgefäss, umfassend einen Detektor (3) zum Erfassen eines elektromagnetischen und/oder eines akustischen Signals, **dadurch gekennzeichnet, dass** die Vorrichtung (1) in einem Prozessgefäss anordenbar ist.

2. Vorrichtung (1) nach Anspruch 1, **dadurch gekennzeichnet, dass** die Vorrichtung (1) zusätzlich eine Quelle (4) zum Erzeugen des elektromagnetischen und/oder des akustischen Signals aufweist.

3. Vorrichtung (1) nach Anspruch 1 oder 2, **dadurch gekennzeichnet, dass** das Signal für das menschliche Auge und/oder das menschliche Gehör nicht wahrnehmbar ist.

4. Vorrichtung (1) nach Anspruch 2 oder 3, **dadurch gekennzeichnet, dass** die Quelle (4) und der Detektor (3) in einer Einheit angeordnet sind.

5. Vorrichtung (1) nach Anspruch 4 **dadurch gekennzeichnet, dass** die Einheit stabförmig ausgestaltet, insbesondere gekapselt in einem für das Signal transparentem Stab, insbesondere einem Glasstab (6) oder einem Kunststoffstab, angeordnet, ist.

6. Vorrichtung (1) nach einem der Ansprüche 2 bis 5, **dadurch gekennzeichnet, dass** die Quelle (4) und der Detektor (3) derart relativ zueinander angeordnet sind, dass der Detektor (3) ein von einem Medium (7) reflektiertes Signal und/oder ein durch das Medium (7) transmittiertes Signal detektiert.

7. Vorrichtung (1) nach einem der Ansprüche 1 bis 6, **dadurch gekennzeichnet, dass** eine Empfangsbegrenzung (17), insbesondere eine Blende, ein Tubus oder eine Blende zum Abschirmen eines unerwünschten Streusignals vor dem Detektor (3) angeordnet ist.

8. Vorrichtung (1) nach einem der Ansprüche 1 bis 7, **dadurch gekennzeichnet, dass** die Vorrichtung (1) zusätzlich eine Auswerteinheit zum Auswerten des vom Detektor (3) erfassten Signals umfasst.

9. Verwendung einer Vorrichtung (1) nach einem der Ansprüche 1 bis 8, **dadurch gekennzeichnet, dass** die Vorrichtung (1) im Prozessgefäss angeordnet ist.

10. Verdampfungssystem (10), insbesondere ein Rotationsverdampfer, umfassend ein Prozessgefäss und eine Vorrichtung (1), insbesondere gemäss einem der Ansprüche 1 bis 8, zur Erkennung einer Schaumentwicklung mit einem Detektor (3) zum Erfassen eines elektromagnetischen und/oder akustischen Signals, **dadurch gekennzeichnet, dass** die Vorrichtung (1) im Prozessgefäss anordenbar oder angeordnet ist.

11. Verdampfungssystem (10) nach Anspruch 10, **dadurch gekennzeichnet, dass** das Verdampfungssystem (10) Mittel aufweist, um Schaum im Prozessgefäss zu reduzieren.

12. Verfahren zum Erkennen einer Schaumentwicklung in einem Prozessgefäss mit einer Vorrichtung (1) zur Schaumerkennung, insbesondere mit einer Vorrichtung (1) gemäss einem der Ansprüche 1 bis 8, oder mit einem Verdampfungssystem (10) gemäss einem der Ansprüche 10 und 11, umfassend die Schritte
- Erfassen eines elektromagnetischen und/oder akustischen Signals mittels eines im Prozessgefäss angeordneten Detektors (3) der Vorrichtung (1), und
- Auswerten des erfassten Signals nach vorgegebenen Kriterien zur Erkennung einer Schaumentwicklung.

13. Verfahren nach Anspruch 12 zusätzlich umfassend den Schritt Aussenden eines elektromagnetischen und/oder akustischen Signals von einer Quelle (4), die im Prozessgefäss angeordnet ist.

14. Verfahren nach Anspruch 13, **dadurch gekennzeichnet, dass** die Quelle (4) ein Signal im Infrarotbereich, insbesondere in einem Wellenlängebereich von 700 bis 1400 nm, und/oder Schallwellen, insbesondere im Frequenzbereich von 20 KHz bis 1 GHz ausstrahlt.

15. Verfahren nach Anspruch 13 oder 14, **dadurch gekennzeichnet, dass** der Detektor (3) das von der Quelle (4) ausgesendete und von Schaum im Prozessgefäss durch Reflexion und/oder Transmission veränderte Signal detektiert.

16. Verfahren nach einem der Ansprüche 12 bis 15 zusätzlich umfassend den Schritt Reduktion eines Schaums bei Anwesenheit von Schaum, insbesondere durch:
- Druckerhöhung im Prozessgefäss und/oder
- Reduktion einer Prozesstemperatur und/oder
- Veränderung der Bewegungsparameter des Prozessgefässes.
